# EUROPÄISCHE PATENTSCHRIFT

(11) **EP 1 523 362 B1**
(45) Veröffentlichungstag und Bekanntmachung des Hinweises auf die Patenterteilung: **04.07.2012**
(21) Anmeldenummer: 03761540.8
(22) Anmeldetag: 27.06.2003
(51) Int. Cl.: A61M 11/00, B05B 17/06, A61M 15/00

(54) **ULTRASCHALLZERSTÄUBER**
ULTRASONIC ATOMIZER
NEBULISEUR ULTRASONIQUE

(30) Priorität: 01.07.2002 DE 10229538
(43) Veröffentlichungstag der Anmeldung: 20.04.2005
(73) Patentinhaber: Johnson Matthey Catalysts (Germany) GmbH, 96257 Redwitz (DE)
(72) Erfinder: KLUMP, Stefan, 96215 Lichtenfels (DE); LINDEN, Klaus van der, 96527 Redwitz-Unterlangenstadt (DE); RÜTTEL, Martin, 96271 Grub am Forst (DE)
(74) Vertreter: FDST Patentanwälte
(86) Internationale Anmeldenummer: PCT/EP2003/006820
(87) Internationale Veröffentlichungsnummer: WO 2004/002558

(56) Entgegenhaltungen:
- DE-A- 2 308 584
- DE-A- 3 826 101
- DE-A- 3 936 248
- DE-A1- 19 520 796
- GB-A- 1 270 700
- US-A- 4 912 357
- US-A- 5 487 378

## Beschreibung

Die Erfindung betrifft einen Ultraschallzerstäuber zur Zerstäubung einer Flüssigkeit.

Bei einer Vielzahl von technischen Anwendungen ist es erforderlich aus Flüssigkeiten Aerosole zu erzeugen. Ultraschallzerstäuber finden ihre Anwendung dabei auf verschiedensten Gebieten. Besonders geeignet sind sie für die Luftbefeuchtung in Kühltheken, in der Medizin für Inhalationsgeräte, oder zum Erzeugen von Klein-Klimata. Ihre Vorteile liegen nicht nur in einem geräuschlosen Betrieb oder platzsparender Bauweise, sondern sind vor allem in punkto Wirtschaftlichkeit und Umweltschutz zu sehen.

Bei einem piezokeramischen Ultraschallzerstäuber werden Flüssigkeiten ohne Druck und Erhitzen nach einem ganz speziellen Verfahren zerstäubt. Ein Piezozerstäuberelement wird mit Flüssigkeit benetzt und durch Ultraschall in Schwingungen versetzt. Dabei wird ein Aerosol erzeugt, das in seiner Homogenität einzigartig ist.

In der EP 0 246 515 A1 ist ein Ultraschall-MHz-Schwinger, insbesondere zur Flüssigkeitsbestäubung, offenbart, bei dem ein Amplitudentransformator auf einer Piezokeramikscheibe befestigt ist, wobei der Amplitudentransformator - ausgehend von der Piezokeramikscheibe - sich zunächst verjüngt und anschließend in einem sich verbreiternden Zerstäuberteller endet. Dieser Zerstäuberteller weist eine konkave Oberfläche ("Hohlspiegel") zur Aufnahme der zu zerstäubenden Flüssigkeit auf. Beim Betrieb eines solchen Ultraschall-Zerstäubers hat sich gezeigt, dass hohe Energieverluste durch die Reflexion der Ultraschallwellen an der Grenzschichtflüssigkeit/Luft bei nicht geeigneter Flüssigkeitshöhe im Zerstäuberteller auftreten.

Aus der EP 0 657 226 B1 ist ein Ultraschallzerstäubergerät zum Zerstäuben einer Flüssigkeit mit einer Piezokeramik bekannt. Ein Gaseinschlüsse aufweisendes elastisches Material dichtet einen Zwischenraum zwischen dem Gehäuseteil und der in die Flüssigkeit abstrahlenden Seite der Piezokeramik gegen die zu zerstäubende Flüssigkeit ab. Dieses elastische, Gaseinschlüsse aufweise Material bewirkt einerseits eine Dichtung gegen die zu zerstäubende Flüssigkeit und andererseits, das der von der Piezokeramik abgestrahlte Ultraschall an den Gaseinschlüssen in dem Material reflektiert wird. Damit kann der im Bereich des Zentrums abgestrahlte Ultraschall nicht über eine den Ultraschall leitende Dichtverbindung in den Gehäuseteil eindringen. Dieser kann damit besonders im Bereich der Öffnung nicht durch den abgestrahlten Ultraschall zerstört werden.

Bei der Ultraschallzerstäubung, insbesondere im Hochfrequenzbereich, ist für die Lebensdauer des Ultraschallzerstäubers besonders die Resistenz gegenüber Kavitationseffekten wichtig. Die Ultraschallkavitation tritt dabei auch in völlig entgasten Flüssigkeiten bei Vorhandensein eines intensiven Schallfeldes auf. Während der negativen Druckphase einer Schallwelle zereisst die Flüssigkeit, und es kommt zur Bildung von Hohlräumen, die in der nachfolgenden Druckphase zusammenstürzen. Die dabei auftretenden Drücke können nach einer Gleichung von Rayleigh bestimmt werden. Die mit einem Zischgeräusch verbundene Ultraschallkavitation führt zu erheblichen Materialzerstörungen und beeinträchtigt daher die Lebensdauer des Ultraschallzerstäubers, insbesondere der Piezokeramik. Ist in der zu zerstäubenden Flüssigkeit gelöstes Gas enthalten, so tritt infolge des durch Ultraschall hervorgerufenen örtlichen Unterdrucks eine Gasabscheidung in Form kleiner Blasen auf. Dieser Vorgang wird als Pseudokavitation bezeichnet.

Durch Kavitation wird demzufolge die mit der zu zerstäubenden Flüssigkeit benetzte Oberfläche des Ultraschallzerstäubers dauerhaft und irreversibel beschädigt und damit die Lebensdauer erheblich reduziert.

Aufgabe der Erfindung ist es daher einen Ultraschallzerstäuber mit einer langen Betriebslebensdauer anzugeben, der insbesondere eine erhöhte Resistenz gegenüber Kavitationseffekten aufgrund des Ultraschallfeldes aufweist.

Die Aufgabe wird erfindungsgemäß gelöst durch einen Ultraschallzerstäuber zur Zerstäubung einer Flüssigkeit nach Anspruch 1.

Die Erfindung geht von der Erkenntnis aus, dass herkömmliche Maßnahmen zum Schutz eines Ultraschallzerstäubers gegenüber Kavitation nur unzureichende Ergebnisse liefern. Die hierzu vorgeschlagenen herkömmlichen Maßnahmen beschränken sich auch beispielsweise auf Schutzüberzüge aus Lack oder Kunststoff. Diese bieten jedoch keine effektive Resistenz gegenüber der Ultraschallkavitation. Darüber hinaus ist auch der Wärmeausdehnungskoeffizient bei derartigen Schutzüberzügen sehr verschieden zu dem Wärmeausdehnungskoeffizient der zu schützenden Piezokeramik. Dadurch entstehen aufgrund des Wärmeeintrags in den Ultraschallzerstäuber thermisch induzierte mechanische Verspannungen, die sich verschlechternd auf die Schutzüberzüge auswirken und diese ablösen. Dieser Effekt wird verstärkt durch die Ultraschallkavitation selbst, so dass die Piezokeramik rasch unbrauchbar wird.

Mit der Erfindung wird nun erstmals ein gegenüber Kavitation hoch resistenter Ultraschallzerstäuber zur Zerstäubung einer Flüssigkeit angegeben. Auf die elektrisch anregbare Piezokeramik ist eine Schutzbeschichtung aufgebracht, welche Aluminiumoxid und/oder Zirkonoxid enthält. Es hat sich überraschender Weise gezeigt, dass eine Schutzbeschichtung, die Aluminiumoxid und/oder Zirkonoxid enthält in hohem Maße Kavitationsresistent ist. Dies ist insbesondere auf die Härte dieser Materialien zurückzuführen.

Von besonderem Vorteil erweist sich auch die hervorragende Wärmeleitfähigkeit von Aluminiumoxid und Zirkonoxid. Dadurch kann im Gegensatz zu herkömmlichen Schutzmaßnahmen ein Wärmestau im Betrieb des Ultraschallzerstäubers sicher vermieden werden, da die Wärme sehr wirksam aus der Schutzbeschichtung abgeführt wird.

Ein weiterer Vorteil ist es, dass Aluminiumoxid und Zirkonoxid einen ähnlichen Wärmeausdehnungskoeffizienten wie die Piezokeramik aufweist. Damit gibt es praktisch keine Verspannungen nach dem Aufbringen der Schutzbeschichtung bei Temperaturänderungen, z.B. wenn sich die Piezokeramik bei einem Trockenlauf stark erhitzt. Somit ist die Aluminiumoxid und/oder Zirkonoxid enthaltende Schutzbeschichtung ein exzellentes Anpassmaterial an die Piezokeramik für die Zerstäubung der Flüssigkeit. Aufgrund der Härte von Aluminiumoxid und Zirkonoxid ist neben der Kavitationsresistenz eine hohe Kratzfestigkeit gegeben, was die Wartung und Reinigung der der Flüssigkeit des Ultraschallzerstäubers ausgesetzten Oberflächen, beispielsweise beim Entfernen von Kalkablagerungen, erleichtert. Ein mechanisches Zerstören der Schutzbeschichtung und damit des Ultraschallzerstäubers selbst ist mit der Schutzbeschichtung der Erfindung praktisch ausgeschlossen.

Die kavitationsresistente Schutzbeschichtung kann dabei auch als Schichtsystem mit mehreren Schichten ausgestaltet sein, wobei zumindest eine der Schichten der Schutzbeschichtung Aluminiumoxid und/oder Zirkonoxid aufweist.

Erfindungsgemäß weist die Schutzbeschichtung eine Aluminiumoxidschicht oder eine Zirkonoxidschicht auf, die auf der in die Flüssigkeit abstrahlende Seite der Piezokeramik aufgebracht ist. Damit wird insbesondere die der zu zerstäubenden Flüssigkeit direkt ausgesetzte Oberfläche des Ultraschallzerstäubers besonders gegen Kavitationseffekte geschützt.

Vorzugsweise ist die Schutzbeschichtung mittels eines Klebstoffs, insbesondere ein Epoxidharzkleber, auf eine der zu zerstäubenden Flüssigkeit zugewandten Oberfläche aufgeklebt.

Die Klebeverbindung ermöglicht ein besonders sicheres und langzeitstabiles Aufbringen der Schutzbeschichtung auf die Oberfläche. Dadurch ist auch eine gute Ankopplung der Schutzbeschichtung an die vor Kavitationseffekten zu schützende Piezokeramik gegeben. Die Oberfläche auf der die Schutzbeschichtung aufgeklebt wird, kann dabei eine Oberfläche der Piezokeramik selbst sein oder aber vorzugsweise eine Oberfläche einer auf der Piezokeramik aufgebrachten elektrisch leitfähigen Kontaktschicht, welche aus als Elektrode zur Einkopplung einer Hochfrequenz dient. Alternativ kann neben einer Klebeverbindung die Ankopplung der Schutzbeschichtung auch über eine Wasserschicht oder eine Ölschicht erfolgen.

Bevorzugt weist die Schutzbeschichtung eine Dicke zwischen 0,01 mm und 10 mm auf. Insbesondere beträgt die Dicke der Schutzbeschichtung 180 µm. Eine solche Schutzbeschichtung wird im Falle einer Klebeverbindung über eine Klebeschicht von ca. 1 µm bis 100 µm, insbesondere 5 µm, mit der elektrisch anregbaren Piezokeramik fest verklebt.

In bevorzugter Ausgestaltung weist der Ultraschallzerstäuber zur Schwingungsanregung der Piezokeramik eine Elektrode auf, über die eine Hochfrequenz einkoppelbar ist. Hierzu sind beispielsweise metallische Hochfrequenzelektroden auf die Piezokeramik aufgebracht. Die Metallisierung kann dabei herstellungstechnisch auf verschiedene Weisen aufgebracht sein. Durch Aufdampfen bzw. Sputtern von reinen Metallen oder Metalllegierungen, durch Aufdrucken von metall- oder kohlenstoffhaltigen leitfähigen Druckpasten, z.B. mittels eines Siebdruckverfahrens mit oder ohne anschließendem Einbrennvorgang. Ebenso kann eine Leitlackbeschichtung mittels eines Sprühvorgangs, eines Druckvorgangs etc. durchgeführt werden, um eine Elektrode auf die Piezokeramik aufzubringen.

Die Elektrode selbst ist dabei bevorzugt als eine Metallelektrode aus Gold, Silber oder Kupfer oder als eine Graphitelektrode ausgestaltet. Beispielsweise wird eine Silberelektrode in Form einer Metallisierung mit einer Dicke von 5 bis 10 µm realisiert. Alternativ kann eine Goldelektrode mit einer Dicke von kleiner als 1 µm vorgesehen sein, über die eine Hochfrequenz zur Anregung der Piezokeramik ankoppelbar ist.

Vorzugsweise ist die Piezokeramik als Piezokeramikscheibe mit einem Durchmesser zwischen 2 mm und 200 mm ausgestaltet.

Die Dicke der Piezokeramikscheibe ist dabei bevorzugt größer als 1 mm, insbesondere zwischen 1 mm und 50 mm.

Der Ultraschallzerstäuber gemäß der Erfindung findet bevorzugt Anwendung in einer Zerstäubervorrichtung zur Erzeugung eines Sprühnebels mit einem Gehäuseteil zur Aufnahme einer zu zerstäubenden Flüssigkeit.

Eine derartige Zerstäubervorrichtung umfasst eine Ultraschall-Flüssigkeitszerstäuber-Einheit mit einer Piezokeramikscheibe, die zu Hochfrequenzschwingungen angeregt wird und unterschiedliche Flüssigkeiten zerstäuben kann. Die zerstäubte Flüssigkeit - auch Aerosol genannt - besteht zum größten Teil aus Tröpfchen mit einem Durchmesser von etwa 15 µm. Mit einer derartigen Zerstäubervorrichtung bestehen gegenüber herkömmlichen Systemen erhebliche Vorteile. Von besonderem Vorteil erweist sich der in der Zerstäubervorrichtung vorgesehene Ultraschallzerstäuber gemäß der Erfindung, dessen Schutzbeschichtung auf der Piezokeramik enthaltend Aluminiumoxid und/oder Zirkonoxid aufgrund der Kavitationsresistenz und der günstigen Anpassung der Wärmeausdehnungskoeffizienten an das Substrat eine deutlich erhöhte Zuverlässigkeit bei gesteigerten Standzeiten aufweist.

Eine derartige Zerstäubervorrichtung ist insbesondere zur Zerstäubung von Wasser, beispielsweise für Luftbefeuchter, Kleinklimakammern, Klimaanlagen, Terrarien und Luftwäscher geeignet.

Die Erfindung wird nachfolgend anhand einer Zeichnung näher erläutert, wobei gleiche Bezugszeichen in den einzelnen Figuren dieselbe Bedeutung haben. Dabei zeigen schematisch und nicht maßstäblich
- FIG 1: einen Ultraschallzerstäuber mit einer Schutzbeschichtung gemäß der Erfindung,
- FIG 2: eine Zerstäubervorrichtung mit einem Ultraschallzerstäuber.

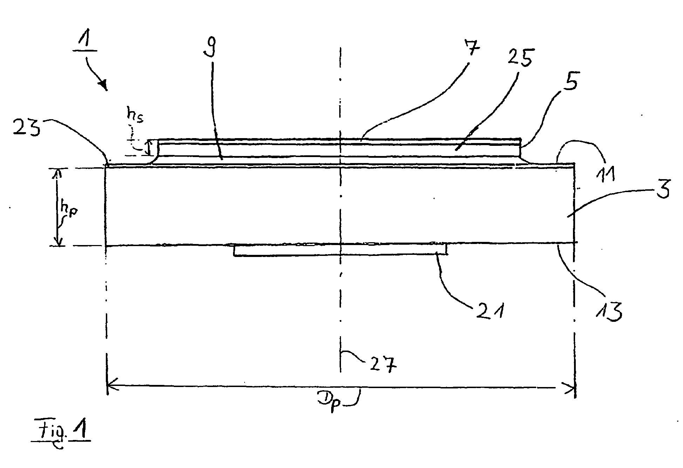

In FIG 1 ist ein Ultraschallzerstäuber 1 in einer Seitenansicht dargestellt. Der Ultraschallzerstäuber 1 ist im Wesentlichen scheibenförmig ausgestaltet mit einer Zylindersymmetrie entlang der Symmetrieachse 27. Der Ultraschallzerstäuber 1 besteht aus einer elektrisch anregbaren Piezokeramik 3, welche als Piezokeramikscheibe 13 mit einem Durchmesser D_{P} ausgestaltet ist. Je nach Anwendungsfall und Einsatz des Ultraschallzerstäubers 1 kann der Durchmesser Dp zwischen etwa 2 mm und 200 mm betragen. Größere Abmessungen sind aber ohne weiteres ebenso realisierbar. Die Dicke h_{P} der Piezokeramikscheibe 13 beträgt zwischen etwa 1,0 mm und 50 mm. Zur Einkopplung einer Hochfrequenz zur elektrischen Anregung der Piezokeramik 3 sind Elektroden 21,23 auf der Piezokeramik 3 aufgebracht. Die Elektrode 21 ist als Silber-Metallisierung mit einer Dicke von 8 µm auf die Unterseite der Piezokeramikscheibe 13 aufgedampft. Auf der entlang der Symmetrieachse 27 der Elektrode 21 gegenüberliegenden Seite der Piezokeramikscheibe 13 ist die Elektrode 23 als dünne Gold-Metallisierung mit einer Dicke von etwa 1 µm auf die Piezokeramik 3 aufgedampft. Das Aufbringen der Edelmetallelektroden 21,23 kann hierbei in einem Sputterprozess erfolgen. Die Elektrode 23 weist eine Oberfläche 11 auf, die im Betrieb des Ultraschallzerstäubers 1 einer in der FIG 1 nicht näher dargestellten zu zerstäubenden Flüssigkeit zugewandt ist.

Um einen wirksamen Schutz vor Ultraschallkavitation für den Ultraschallzerstäuber 1, insbesondere für die Piezokeramik 3 zu erreichen, ist auf der Piezokeramik 3 eine Schutzbeschichtung 5 enthaltend Aluminiumoxid und/oder Zirkonoxid aufgebracht. Dabei ist die Schutzbeschichtung 5 mittels eines Klebstoffs 9 auf die Oberfläche 11 aufgeklebt. Als Klebstoff 9 kommt beispielsweise ein Epoxidharzkleber zum Einsatz, der eine Klebeschicht mit einer Dicke von etwa 5 µm bildet.

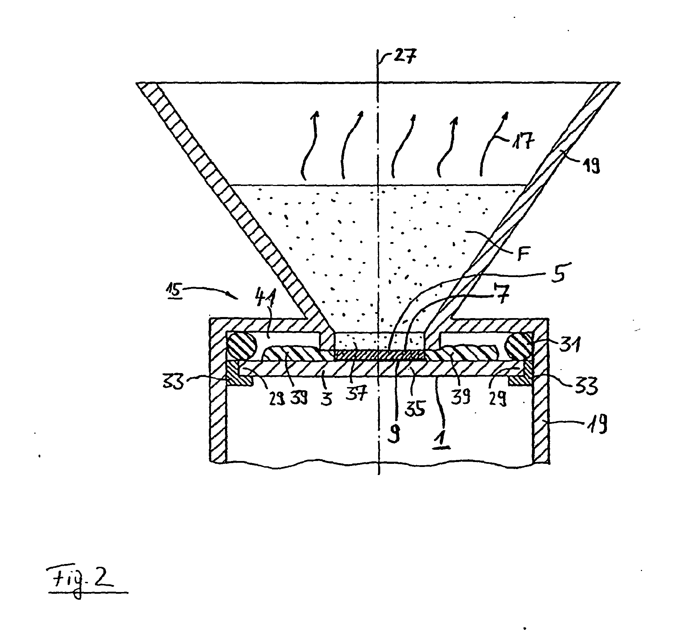

Als äußere Schicht weist die Schutzbeschichtung 5 eine Aluminiumoxidschicht 7 auf, die auf der in die Flüssigkeit F (vgl. FIG 2) abstrahlende Seite der Piezokeramik 3 aufgebracht ist. In alternativer Ausgestaltung kann die Schicht 7 auch eine Zirkonoxidschicht sein. Neben der Aluminiumoxidschicht 7 weist die Schutzbeschichtung 5 eine Zwischenschicht 25 auf, welche an den Klebstoff 9 sowie an die Aluminiumoxidschicht 7 angrenzt. Die Zwischenschicht 25 ist optional und dient insbesondere einer weiteren Anpassung der Wärmeausdehnungskoeffizienten zwischen der Aluminiumoxidschicht 7 und der Piezokeramik 3. Im einfachsten Fall kann die Zwischenschicht 25 ebenfalls aus Aluminiumoxid bestehen. Hierbei kann die Schutzbeschichtung 5 als dünne Scheibe aus Aluminiumoxid ausgestaltet sein, die mittels des Klebers 9 auf die Oberfläche 11 aufgelebt wird. Somit erfolgt im Betrieb ein Kontakt zu der zu zerstäubenden Flüssigkeit 11 nur über die Aluminiumoxidschicht 7, so dass die Piezokeramik 3 gut geschützt ist. Überraschenderweise konnte gefunden werden, dass insbesondere Aluminiumoxid für die Zwecke des Schutzes vor Ultraschallkavitation hervorragend geeignet ist. Aufgrund der Härte von Aluminiumoxid ist dieses Material offenbar besonders Kavitationsresistent. Überdies ist Aluminiumoxid und/oder Zirkonoxid günstig aufgrund der hohen Wärmeleitfähigkeit, so dass bei einer elektrischen Anregung der Piezokeramik 3 ein Wärmestau innerhalb der Piezokeramik 3 sicher vermieden ist. Von besonderem Vorteil erweist sich, dass Aluminiumoxid einen ähnlichen Wärmeausdehnungskoeffizienten wie die Piezokeramik 3 aufweist, so dass es keine Verspannungen nach dem Verkleben der Aluminiumoxidschicht 7 oder bei einer Temperaturänderung, z.B. bei einem Trockenlauf des Ultraschallzerstäubers 1, zu verzeichnen gibt. Aufgrund der Härte von Aluminiumoxid und Zirkonoxid ist weiterhin eine hohe Kratzfestigkeit der von der Schutzbeschichtung 5 gebildeten äußeren Oberfläche gegeben. Bei einer Reinigung des Ultraschallzerstäubers von Ablagerungen wird somit die Schutzbeschichtung 5 nicht verkratzt, so dass diese sehr langlebig ist.

Ein weiteres Ausführungsbeispiel der Erfindung ist in FIG 2 dargestellt. Dabei zeigt FIG 2 schematisch einen Längsschnitt eines Oberteils einer hier nicht näher dargestellten Zerstäubervorrichtung 15. In dem dargestellten Längsschnitt erkennt man einen achsensymmetrischen Aufbau der Zerstäubervorrichtung 15 zu einer eingezeichneten Symmetrieachse 27, welche eine Zylindersymmetrie offenbart. Ein Ultraschallzerstäuber 1 ist in einem Gehäuseteil 19 angeordnet, welches zur Aufnahme einer zu zerstäubenden Flüssigkeit F trichterförmig ausgestaltet ist. In einem Randbereich 29 der Piezokeramik 3 ist ein O-Ring 31 aus Kunststoffvollmaterial angeordnet, der in diesem Betreich den Ultraschallzerstäuber 1 gegen den Gehäuseteil 19 abdichtet. In diesem Bereich wird der Ultraschallzerstäuber 1 mittels eines Gegenlagers (Sprengring) 33 gehalten und zugleich positioniert. Im Bereich des Strahlungszentrums 35 der Piezokeramik 3 weist das Gehäuseteil 19 eine im Ausführungsbeispiel kreisrunde Öffnung 37 auf, durch die zu erstäubende Flüssigkeit F mit einer Oberfläche des Ultraschallzerstäubers 1 wechselwirkt. Diese äußere Oberfläche des Ultraschallzerstäubers 1 ist durch eine Aluminiumoxidschicht 7 gebildet, die mittels des Klebers 9 mit der elektrisch anregbaren Piezokeramik 3 fest verbunden ist. Die elektrische Anregung der Piezokeramik 3 erfolgt über ein Hochfrequenzfeld, welches mittels in der FIG 2 nicht näher dargestellten Elektroden eingekoppelt wird. Im Bereich der Ränder der Öffnung 37 wird ein Zwischenraum 41 zwischen dem Ultraschallzerstäuber 1 und dem Gehäuseteil 19 von einem Silikonschaum 39 abgedichtet.

Dieses elastische, Gaseinschlüsse aufweisende Material 39 bewirkt einerseits eine Dichtung gegen die zu zerstäubende Flüssigkeit F und andererseits, dass der von der Piezokeramik 3 abgestrahlte Ultraschall an den Gaseinschlüssen im Silikonschaum 39 reflektiert wird. Damit kann der im Bereich des Strahlungszentrums 35 abgestrahlte Ultraschall nicht über eine den Ultraschall leitende Dichtverbindung in den Gehäuseteil eindringen. Dieser kann damit besonders im Bereich der Öffnung 37 nicht durch den abgestrahlten Ultraschall zerstört werden.

Alternativ zu dem hier dargestellten Ausführungsbeispiel kann der Zwischenraum 41, der von dem Ultraschallzerstäuber 1, dem Silikonschaum 39, dem O-Ring 31 und dem Gehäuseteil 19 eingeschlossen wird, ganz mit Silikonschaum 39 ausgefüllt sein. Auf diese Weise wird erreicht, dass bei nicht idealer Dichtwirkung des Silikonschaums 39 keine Volumina zur Ansammlung von Flüssigkeit und Bakterien im Zwischenraum 41 übrig bleiben.

Im Betrieb der Zerstäubungsvorrichtung 15 wird der Ultraschallzerstäuber 1 durch eine Hochfrequenzeinkopplung aktiviert. Dabei steht die zu zerstäubende Flüssigkeit F in Kontakt mit dem Ultraschallzerstäuber 1. Der Kontakt erfolgt über die Aluminiumoxidschicht 7, welche aufgrund der Materialwahl eine besonders hohe Kavitationsresistenz aufweist. Hierdurch ist der Ultraschallzerstäuber 1 gegen Zerstörung durch Kavitationseffekte sicher geschützt, wobei insbesondere die Piezokeramik 3 keine Schäden erfahren kann. Somit ist ein besonders langlebiger und besonders zuverlässiger Ultraschallzerstäuber 1 gegeben, der in der Zerstäubervorrichtung 15 problemlos zum Einsatz kommen kann. Die Schutzbeschichtung 5 kann neben einer Aluminiumoxidschicht 7 auch weitere Schichten mit unterschiedlicher Zusammensetzung aufweisen. Von besonderer Bedeutung ist, dass aufgrund der Materialwahl die mit der Flüssigkeit F in Kontakt stehende Oberfläche des Ultraschallzerstäubers 1 durch ein gegenüber Ultraschallkavitation resistentes Material gebildet ist. Vorteilhafterweise erfolgt die Materialwahl zusätzlich im Hinblick auf eine möglichst gute Anpassung an den Wärmeausdehnungskoeffizienten der durch die Schutzbeschichtung 5 zu schützenden Piezokeramik 3. Besonders günstig im Hinblick auf diese Anforderungen hat sich eine Schutzbeschichtung 5 auf der Piezokeramik 3 erwiesen, die Aluminiumoxid und/oder Zirkonoxid enthält.

Mit der Zerstäubervorrichtung 15 ist mit Flüssigkeit F ein Sprühnebel 17 erzeugbar, wie er für medizinische Anwendungen, zur Luftbefeuchtung, und anderen Applikationen besonders vorteilhaft eingesetzt werden kann.

## Patentansprüche

1. Ultraschallzerstäuber (1) zur Zerstäubung einer Flüssigkeit (F), mit einer elektrisch anregbaren Piezokeramik (3)
**dadurch gekennzeichnet, dass** auf der in die Flüssigkeit (F) abstrahlenden Seite der Piezokeramik (3) eine Schutzbeschichtung (5) aufgebracht ist, die
eine Aluminiumoxidschicht (7) oder eine Zirkonoxidschicht aufweist, so dass im Betrieb ein Kontakt zu der zu zerstäubenden Flüssigkeit (7) nur über die Aluminiumoxidschicht (7) bzw. Zirkonoxidschicht erfolgt.

2. Ultraschallzerstäuber (1) nach Anspruch 1
**dadurch gekennzeichnet, dass** die Schutzbeschichtung (5) mittels eines Klebstoffs (9), insbesondere ein Epoxidharzkleber, auf eine der zu zerstäubenden Flüssigkeit (F) zugewandten Oberfläche (11) aufgeklebt ist.

3. Ultraschallzerstäuber (1) nach Anspruch 1 oder 2, **dadurch gekennzeichnet, dass** die
Schutzbeschichtung (5) eine Dicke (hₛ) zwischen 0,01 mm und 10,0 mm, insbesondere 180 µm, aufweist.

4. Ultraschallzerstäuber (1) nach einem der vorhergehenden Ansprüche,
**dadurch gekennzeichnet, dass** dieser zur Schwingungsanregung der Piezokeramik (3) eine Elektrode (21, 23) aufweist, über die eine Hochfrequenz einkoppelbar ist.

5. Ultraschallzerstäuber (19) nach Anspruch 4,
**dadurch gekennzeichnet, dass** die Elektrode (21, 23) als eine Metallelektrode aus Gold, Silber oder Kupfer oder als eine Graphitelektrode ausgestaltet ist.

6. Ultraschallzerstäuber (1) nach einem der vorhergehenden Ansprüche,
**dadurch gekennzeichnet, dass** die Piezokeramik (3) als Piezokeramikscheibe (13) mit einem Durchmesser (D_{P}) zwischen 2 mm und 200 mm ausgestaltet ist.

7. Ultraschallzerstäuber (1) nach Anspruch 6,
**dadurch gekennzeichnet, dass** die Piezokeramikscheibe (13) eine Dicke (hₚ) zwischen 1,0 mm und 50 mm aufweist.

8. Zerstäubervorrichtung (15) zur Erzeugung eines Sprühnebels (17) mit einem Gehäuseteil (19) zur Aufnahme einer zu zerstäubenden Flüssigkeit (F) und einem Ultraschallzerstäuber (1) nach einem der vorhergehenden Ansprüche.

## Claims

1. Ultrasonic atomizer (1) for atomizing a liquid (F), having an electrically excitable piezoceramic element (3), **characterised in that** a protective coating (5) is applied to the side of the piezoceramic element (3) radiating into the liquid (F), said protective coating comprising an aluminium oxide layer (7) or a zirconium oxide layer so that, during operation, contact with the liquid (F) to be atomized only occurs via the aluminium oxide layer (7) and/or zirconium oxide layer.

2. Ultrasonic atomizer (1) according to claim 1, **characterised in that** the protective coating (5) is adhesively bonded by means of an adhesive (9), in particular an epoxy resin adhesive, to a surface (11) facing the liquid (F) to be atomised.

3. Ultrasonic atomizer (1) according to claim 1 or 2, **characterised in that** the protective coating (5) has a thickness (hₛ) between 0.01 mm and 10.0 mm, in particular 180 µm.

4. Ultrasonic atomizer (1) according to one of the preceding claims, **characterised in that** it comprises an electrode (21, 23) for vibrational excitation of the piezoceramic element (3), it being possible to couple in a high frequency via said electrode.

5. Ultrasonic atomizer (19) according to claim 4, **characterised in that** the electrode (21, 23) is formed as a metal electrode made of gold, silver or copper, or as a graphite electrode.

6. Ultrasonic atomizer (1) according to one of the preceding claims, **characterised in that** the piezoceramic element (3) is formed as a piezoceramic disc (13) having a diameter (Dₚ) between 2 mm and 200 mm.

7. Ultrasonic atomizer (1) according to claim 6, **characterised in that** the piezoceramic disc (13) has a thickness (hₚ) between 1.0 mm and 50 mm.

8. Atomiser device (15) for producing a spray mist (17), having a housing part (19) for holding a liquid (F) to be atomized and having an ultrasonic atomizer (1) according to one of the preceding claims.

## Revendications

1. Nébuliseur ultrasonique (1) pour la nébulisation d'un liquide (F), comprenant une céramique piézoélectrique (3) pouvant être excitée par voie électrique, **caractérisé en ce que** sur la face de la céramique piézoélectrique (3), qui rayonne dans le liquide (F), est appliqué un revêtement de protection (5) comprenant une couche d'oxyde d'aluminium (7) ou une couche d'oxyde de zirconium, de sorte qu'en fonctionnement, il ne se produit un contact avec le liquide (F) à nébuliser que par l'intermédiaire de la couche d'oxyde d'aluminium (7) ou respectivement de la couche d'oxyde de zirconium.

2. Nébuliseur ultrasonique (1) selon la revendication 1, **caractérisé en ce que** le revêtement de protection (5) est collé sur une surface (11) dirigée vers le liquide (F) à nébuliser, au moyen d'une colle (9), notamment d'une colle à résine époxy.

3. Nébuliseur ultrasonique (1) selon la revendication 1 ou la revendication 2, **caractérisé en ce que** le revêtement de protection (5) présente une épaisseur (hₛ) comprise entre 0,01 mm et 10,0 mm, notamment de 180 µm.

4. Nébuliseur ultrasonique (1) selon l'une des revendications précédentes, **caractérisé en ce que** celui-ci comporte pour l'excitation vibratoire de la céramique piézoélectrique (3), une électrode (21, 23) par laquelle il est possible d'y induire une haute fréquence.

5. Nébuliseur ultrasonique (1) selon la revendication 4, **caractérisé en ce que** l'électrode (21, 23) est réalisée sous la forme d'une électrode métallique en or, argent ou cuivre, ou sous la forme d'une électrode de graphite.

6. Nébuliseur ultrasonique (1) selon l'une des revendications précédentes, **caractérisé en ce que** la céramique piézoélectrique (3) est réalisée sous la forme d'un disque de céramique piézoélectrique (13) d'un diamètre (Dₚ) compris entre 2 mm et 200 mm.

7. Nébuliseur ultrasonique (1) selon la revendication 6, **caractérisé en ce que** le disque de céramique piézoélectrique (13) présente une épaisseur (hₚ) comprise entre 1,0 mm et 50 mm.

8. Dispositif de nébulisation (15) destiné à produire un nébulisat (17), comprenant une partie de carter (19) prévu pour recevoir un liquide (F) à nébuliser, et un nébuliseur ultrasonique (1) selon l'une des revendications précédentes.
